# FASCICULE DE BREVET EUROPEEN

(11) **EP 3 471 691 B1**
(45) Date de publication et mention de la délivrance du brevet: **11.08.2021**
(21) Numéro de dépôt: 17745378.4
(22) Date de dépôt: 14.06.2017
(51) Int. Cl.: A61K 8/33, A61K 8/35, A61Q 13/00, C11B 9/00, A61P 13/00

(54) **MÉLANGE COMPRENANT AU MOINS DE LA DIHYDRO-5-PENTYL-2(3H)-FURANONE ET DU 2,4-DIMÉTHYL-4-PHÉNYLTÉTRAHYDROFURANE ET SON UTILISATION POUR MASQUER LES MAUVAISES ODEURS**
MISCHUNG MIT MINDESTENS DIHYDRO-5-PENTYL-2(3H)-FURANON UND 2,4-DIMETHYL-4-PHENYLTETRAHYDROFURAN UND VERWENDUNG DAVON ZUR MASKIERUNG UNANGENEHMER GERÜCHE
MIXTURE COMPRISING AT LEAST DIHYDRO-5-PENTYL-2(3H)-FURANONE AND 2,4-DIMETHYL-4-PHENYLTETRAHYDROFURAN AND USE THEREOF FOR MASKING UNPLEASANT ODOURS

(30) Priorité: 16.06.2016 FR 1655608
(43) Date de publication de la demande: 24.04.2019
(73) Titulaire: Expressions Parfumees, 06130 Grasse (FR)
(72) Inventeur: MARIN, Christophe, 06200 Nice (FR); COEZ, Stéphane, 06530 Peymeinade (FR); BUZZI, Jennifer, 06130 Grasse (FR); DEROO, Christophe, 06600 Antibes (FR)
(74) Mandataire: Brizio Delaporte, Allison
(86) Numéro de dépôt international: PCT/FR2017/051526
(87) Numéro de publication internationale: WO 2017/216478

(56) Documents cités:
- EP-B1- 1 737 937
- WO-A2-2008/104352
- CN-A- 1 948 447
- GB-A- 2 528 480

## Description

La présente invention a pour objet un mélange comprenant au moins de la dihydro-5-pentyl-2(3H)-furanone et du 2,4-diméthyl-4-phényltétrahydrofurane, et son utilisation pour masquer les mauvaises odeurs.

Chez l'être humain, le système olfactif est constitué d'un ensemble de capteurs. Une odeur est perceptible dès lors qu'elle atteint un seuil de perception olfactif. Le seuil de perception olfactif correspond à une concentration de molécules odorantes par litre d'air. On parle aussi de seuil de discernement atteint lorsque qu'un individu est en mesure de différencier, discriminer, juger et apprécier une odeur. Ainsi, dans un échantillon, la concentration des molécules odorantes joue un rôle important pour la perception des odeurs.

Lors de l'olfaction, les molécules odorantes remontent la cavité nasale pour atteindre l'épithélium olfactif où se trouvent les cellules nerveuses olfactives. Les molécules odorantes sont captées par les cellules nerveuses olfactives pour engendrer une cascade de réactions aboutissant à la formation d'un message nerveux. Au niveau du système nerveux central, le bulbe olfactif reçoit les messages nerveux et permet leur acheminement jusqu'au cortex olfactif où ils seront identifiés et associés à des valeurs olfactives.

La perception olfactive permet de caractériser les odeurs par leur intensité, leur qualité ainsi que leur appréciation. L'intensité d'une odeur se définit par la quantité de molécules odorantes. La qualité des odeurs est liée à l'identité de la molécule odorante. L'appréciation d'une odeur renvoie au caractère plaisant ou déplaisant d'une sensation olfactive.

Les mélanges de molécules odorantes, aussi bien d'origine naturelle que synthétique, peuvent être utilisés en cosmétique, en parfumerie, ou encore pour les produits ménagers. Ces mélanges permettent d'apporter une composante olfactive agréable à l'utilisateur. Pour certaines applications, cette composante olfactive agréable peut être perturbée par d'autres composantes olfactives désagréables comme les principes actifs du produit contenant ledit mélange odorant ou bien par un facteur extérieur (odeur de tabac, odeur corporelle etc.).

La composante olfactive dite agréable doit donc être suffisamment intense pour compenser la composante olfactive dite désagréable. Trouver l'équilibre entre les composantes olfactives agréables et désagréables constitue donc un problème majeur dans l'industrie de parfumerie.

De plus, pour toute utilisation ultérieure, les mélanges odorants doivent être stables, c'est-à-dire ne pas se dégrader avec le temps et ne pas réagir avec les autres composés du produit qui les contient. EP1737937 divulgue les compositions de parfum qui ont la capacité de réduire ou de prévenir les mauvaises odeurs, c'est-à-dire les odeurs généralement considérées comme désagréables ou indésirables

En parfumerie, l'obtention d'un mélange odorant polyvalent en terme d'applications, qui soit stable, agréable et permette de masquer les mauvaises odeurs, fait l'objet de recherches permanentes.

Les inventeurs ont développé un mélange comprenant au moins de la dihydro-5-pentyl-2(3H)-furanone et du 2,4-diméthyl-4-phényltétrahydrofurane. L'objet de la présente invention permet d'obtenir un mélange odorant avec une intensité et une longévité améliorées capable de masquer les mauvaises odeurs, ledit mélange pouvant être intégré dans n'importe quel type de parfum, i.e. dans n'importe quelle famille olfactive.

La présente invention a ainsi pour objet un mélange comprenant, préférentiellement constitué, de la dihydro-5-pentyl-2(3H)-furanone (gamma-nonalactone) à un pourcentage compris entre 20 et 80% par rapport au poids total dudit mélange et du 2,4-diméthyl-4-phényltétrahydrofurane (rhubafurane) à un pourcentage compris entre 20 et 80% par rapport au poids total dudit mélange.

La présente invention a également pour objet un mélange comprenant, préférentiellement constitué, de la dihydro-5-pentyl-2(3H)-furanone à un pourcentage compris entre 20 et 75% par rapport au poids total dudit mélange et du 2,4-diméthyl-4-phényltétrahydrofurane à un pourcentage compris entre 20 et 75% par rapport au poids total dudit mélange.

Selon l'invention, par « mélange », « mélange masquant » ou « mélange odorant » on entend un mélange comprenant au moins la dihydro-5-pentyl-2(3H)-furanone et le 2,4-diméthyl-4-phényltétrahydrofurane. Le mélange selon la présente invention peut en outre comprendre de l'ester éthylique de l'acide 10-undécénoïque (undécylenate d'éthyle) et/ou de la (3E)-4-(2,6,6-triméthylcyclohex-2-èn-1-yl)but-3-èn-2-one (alpha-ionone). Au sens de la présente invention, on utilisera indifféremment « mélange », « mélange masquant » et « mélange odorant ».

La dihydro-5-pentyl-2(3H)-furanone et le 2,4-diméthyl-4-phényltétrahydrofurane peuvent être utilisés dans des proportions qui varient en fonction de l'usage souhaité.

De manière préférée, la dihydro-5-pentyl-2(3H)-furanone représente dans le mélange selon l'invention entre 30 et 35% par rapport au poids total dudit mélange et le 2,4-diméthyl-4-phényltétrahydrofurane représente dans le mélange selon l'invention entre 30 et 35% par rapport au poids total dudit mélange.

Dans un mode de réalisation préféré, le mélange selon l'invention comprend en outre de l'ester éthylique de l'acide 10-undécénoïque et/ou de la (3E)-4-(2,6,6-triméthylcyclohex-2-èn-1-yl)but-3-èn-2-one.

Dans un mode de réalisation particulier, ledit mélange est constitué de la dihydro-5-pentyl-2(3H)-furanone, du 2,4-diméthyl-4-phényltétrahydrofurane, de l'ester éthylique de l'acide 10-undécénoïque et de la (3E)-4-(2,6,6-triméthylcyclohex-2-èn-1-yl)but-3-èn-2-one.

L'ester éthylique de l'acide 10-undécénoïque et la (3E)-4-(2,6,6-triméthylcyclohex-2-èn-1-yl)but-3-èn-2-one peuvent être utilisés dans des proportions qui varient en fonction de l'usage souhaité.

De manière préférée, la concentration d'ester éthylique de l'acide 10-undécénoïque dans le mélange selon l'invention est comprise entre 20 et 75% par rapport au poids total dudit mélange et la concentration de la (3E)-4-(2,6,6-triméthylcyclohex-2-èn-1-yl)but-3-èn-2-one dans le mélange selon l'invention est comprise entre 20 et 75% par rapport au poids total dudit mélange.

De manière encore plus préférée, la concentration d'ester éthylique de l'acide 10-undécénoïque dans le mélange selon l'invention est comprise entre 30 et 35% par rapport au poids total dudit mélange et la concentration de la (3E)-4-(2,6,6-triméthylcyclohex-2-èn-1-yl)but-3-èn-2-one dans le mélange selon l'invention est comprise entre 30 et 35% par rapport au poids total dudit mélange.

Dans un mode de réalisation particulier, le mélange selon l'invention comprend 35% de dihydro-5-pentyl-2(3H)-furanone par rapport au poids total dudit mélange, 35% de 2,4-diméthyl-4-phényltétrahydrofurane par rapport au poids total dudit mélange et 30% d'ester éthylique de l'acide 10-undécénoïque par rapport au poids total dudit mélange.

Dans un autre mode de réalisation particulier, le mélange selon l'invention comprend 50% de dihydro-5-pentyl-2(3H)-furanone par rapport au poids total dudit mélange et 50% de 2,4-diméthyl-4-phényltétrahydrofurane par rapport au poids total dudit mélange.

Dans un autre mode de réalisation particulier, le mélange selon l'invention comprend 75% de dihydro-5-pentyl-2(3H)-furanone par rapport au poids total dudit mélange et 25% de 2,4-diméthyl-4-phényltétrahydrofurane par rapport au poids total dudit mélange.

Dans un autre mode de réalisation particulier, le mélange selon l'invention comprend 35% de dihydro-5-pentyl-2(3H)-furanone par rapport au poids total dudit mélange, 35% de 2,4-diméthyl-4-phényltétrahydrofurane par rapport au poids total dudit mélange et 30% de (3E)-4-(2,6,6-triméthylcyclohex-2-èn-1-yl)but-3-èn-2-one par rapport au poids total dudit mélange.

Dans un autre mode de réalisation particulier, le mélange selon l'invention comprend 60% de dihydro-5-pentyl-2(3H)-furanone par rapport au poids total dudit mélange, 20% de 2,4-diméthyl-4-phényltétrahydrofurane par rapport au poids total dudit mélange et 20% de (3E)-4-(2,6,6-triméthylcyclohex-2-èn-1-yl)but-3-èn-2-one par rapport au poids total dudit mélange.

Dans un autre mode de réalisation particulier, le mélange selon l'invention comprend 30% de dihydro-5-pentyl-2(3H)-furanone par rapport au poids total dudit mélange, 30% de 2,4-diméthyl-4-phényltétrahydrofurane par rapport au poids total dudit mélange, 20% d'ester éthylique de l'acide 10-undécénoïque par rapport au poids total dudit mélange et 20% de (3E)-4-(2,6,6-triméthylcyclohex-2-èn-1-yl)but-3-èn-2-one par rapport au poids total dudit mélange.

La présente invention a également pour objet une composition qui comprend le mélange selon l'invention.

Ainsi par « composition », on entend une composition comprenant le mélange selon l'invention. Typiquement, cette composition est une composition parfumée.

Préférentiellement, la composition selon l'invention comprend entre 0,5 et 50%, de préférence entre 0,5 et 5% de mélange selon l'invention, par rapport au poids total de ladite composition.

La présente invention a ainsi pour objet une composition comprenant de la dihydro-5-pentyl-2(3H)-furanone à un pourcentage compris entre 0,1 et 15%, de préférence entre 0,1 et 10%, de préférence entre 0,1 et 3,75% par rapport au poids total de ladite composition et du 2,4-diméthyl-4-phényltétrahydrofurane à un pourcentage compris entre 0,1 et 15%, de préférence entre 0,1 et 10%, de préférence entre 0,1 et 3,75% par rapport au poids total de ladite composition.

La dihydro-5-pentyl-2(3H)-furanone peut être utilisée dans des proportions qui varient en fonction de l'usage souhaité.

De manière préférée, la dihydro-5-pentyl-2(3H)-furanone est présente dans la composition selon l'invention à une concentration comprise entre 0,1 et 15%, préférentiellement entre 0,1 et 3,75%, préférentiellement entre 0,1 et 0,75%, plus préférentiellement entre 0,2 et 0,75% et encore plus préférentiellement entre 0,2 et 0,375% par rapport au poids total de ladite composition.

Le 2,4-diméthyl-4-phényltétrahydrofurane peut être utilisé dans des proportions qui varient en fonction de l'usage souhaité.

De manière préférée, le 2,4-diméthyl-4-phényltétrahydrofurane est présent dans la composition selon l'invention à une concentration comprise entre 0,1 et 15%, préférentiellement entre 0,1 et 3,75%, préférentiellement entre 0,1 et 0,75%, plus préférentiellement entre 0,2 et 0,75% et encore plus préférentiellement entre 0,2 et 0,375% par rapport au poids total de ladite composition.

Dans un mode de réalisation particulier, la composition selon l'invention comprend en outre de l'ester éthylique de l'acide 10-undécénoïque et/ou de la (3E)-4-(2,6,6-triméthylcyclohex-2-èn-1-yl)but-3-èn-2-one.

L'ester éthylique de l'acide 10-undécénoïque peut être utilisé dans des proportions qui varient en fonction de l'usage souhaité.

De manière préférée, l'ester éthylique de l'acide 10-undécénoïque est présent dans la composition selon l'invention à une concentration comprise entre 0,1 et 3,75%, préférentiellement entre 0,1 et 0,75%, plus préférentiellement entre 0,2 et 0,75% et encore plus préférentiellement entre 0,2 et 0,375% par rapport au poids total de ladite composition.

La (3E)-4-(2,6,6-triméthylcyclohex-2-èn-1-yl)but-3-èn-2-one peut être utilisée dans des proportions qui varient en fonction de l'usage souhaité.

De manière préférée, la (3E)-4-(2,6,6-triméthylcyclohex-2-èn-1-yl)but-3-èn-2-one est présente dans la composition selon l'invention à une concentration comprise entre 0,1 et 3,75%, préférentiellement entre 0,1 et 0,75%, plus préférentiellement entre 0,2 et 0,75% et encore plus préférentiellement entre 0,2 et 0,375% par rapport au poids total de ladite composition.

Dans un mode de réalisation particulier, la composition selon l'invention comprend entre 0,35 et 5,5% de dihydro-5-pentyl-2(3H)-furanone par rapport au poids total de ladite composition, 0,35% de 2,4-diméthyl-4-phényltétrahydrofurane par rapport au poids total de ladite composition et 0,30% d'ester éthylique de l'acide 10-undécénoïque par rapport au poids total de ladite composition.

Dans un autre mode de réalisation particulier, la composition selon l'invention comprend 0,50% de dihydro-5-pentyl-2(3H)-furanone par rapport au poids total de ladite composition et 0,50% de 2,4-diméthyl-4-phényltétrahydrofurane par rapport au poids total de ladite composition.

Dans un autre mode de réalisation particulier, la composition selon l'invention comprend 0,75% de dihydro-5-pentyl-2(3H)-furanone par rapport au poids total de ladite composition et 0,25% de 2,4-diméthyl-4-phényltétrahydrofurane par rapport au poids total de ladite composition.

Dans un autre mode de réalisation particulier, la composition selon l'invention comprend entre 0,35 et 0,45% de dihydro-5-pentyl-2(3H)-furanone par rapport au poids total de ladite composition, 0,35% de 2,4-diméthyl-4-phényltétrahydrofurane par rapport au poids total de ladite composition et 0,30% de (3E)-4-(2,6,6-triméthylcyclohex-2-èn-1-yl)but-3-èn-2-one par rapport au poids total de ladite composition.

Dans un autre mode de réalisation particulier, la composition selon l'invention comprend 0,60% de dihydro-5-pentyl-2(3H)-furanone par rapport au poids total de ladite composition, 0,20% de 2,4-diméthyl-4-phényltétrahydrofurane par rapport au poids total de ladite composition et 0,20% de (3E)-4-(2,6,6-triméthylcyclohex-2-èn-1-yl)but-3-èn-2-one par rapport au poids total de ladite composition.

Dans un autre mode de réalisation particulier, la composition selon l'invention comprend 0,30% de dihydro-5-pentyl-2(3H)-furanone par rapport au poids total de ladite composition, 0,30% de 2,4-diméthyl-4-phényltétrahydrofurane par rapport au poids total de ladite composition, 0,20% d'ester éthylique de l'acide 10-undécénoïque par rapport au poids total de ladite composition et 0,20% de (3E)-4-(2,6,6-triméthylcyclohex-2-èn-1-yl)but-3-èn-2-one par rapport au poids total de ladite composition.

Dans un mode de réalisation particulier, la composition selon l'invention est une composition parfumée.

Par « composition parfumée », on entend une composition comprenant un mélange de substances parfumantes, tant à l'état isolé qu'en solution ou en suspension, dans leurs diluants, dissolvants ou co-ingrédients habituels. Une telle composition est destinée à apporter une composante olfactive agréable.

Une telle composition parfumée peut comprendre des esters, éthers, alcools, aldéhydes, cétones, lactones, acétals, nitriles, phénols, acides, terpènes , composés hétérocycliques azotés ou soufrés, saturés ou insaturés, et des produits d'origine naturelle.

Des exemples d'esters comprennent, mais ne sont pas limités à, l'acétate de benzyle, acétate de p-tert-butylcyclohexyle, acétate de linalyle, acétate de diméthyl-benzyl-carbinyle, acétate de phényléthyle, acétate de 1,1-diméthyl-2-phényléthyle, benzoate de linalyle, glycinate d'éthyl-méthyl-phényle, propionate d'allylcyclohexyle, propionate de styrallyle, salicylate de benzyle, acétate de méthyl-3-oxo-2-pentylcyclopentane, acétate de prop-2-ènyle-2,3-méthylbutoxy (glycolate d'allyl amyle, ester 2-propénylique de l'acide 3-méthylbutoxy-acétique), ester phénylméthylique de l'acide acétique, acétate d'isoamyle (acétate d'isopentyle), acétate de cis-hex-3-ènyle (acétate de (Z)-hex-3-ènyle), acétate de citronellyle (acétate de 3,7-dimethyl-6-octèn-1-ol), acétate d'hexyle, acétate d'isobornyle (exo-acétate de bicyclo[2.2.1]heptan-2-ol,1,7,7-triméthyle), acétate de méthanyle (acétate d'alpha,alpha,4-triméthylcyclohexylméthyle), acétate d'éthyle, acétate de prényle (acétate de 3-méthyl-2-butényle), acétate de terpényle (acétate de 4-méthyl-1-propan-2-yl-1-cyclohex-2-ènyle), formiate d'alpha-3,3-triméthylcyclohexyle-méthyle, butanoate de 3-méthylbutyle (butyrate d'iso amyle), butanoate d'alpha,alpha-diméthylphenethyl, acétate de 3-dihydrodicyclopentadièn-2,3-yle, prop-2-ènyl, propanoate de 3-cyclohexyle (cyclo hexane propionate d'allyl), heptanoate d'allyle (heptanoate de 2-propényl), 2-methylpropanoate de 2-phénoxy-éthyle (isobutyrate de phénoxy éthyle), 2-méthyl-pentanoate d'éthyle, 2-méthyl-butyrate d'éthyle (ester éthylique de l'acide 2-méthyl-butanoïque), 1,4-dioxacycloheptadecane-5,17-dione (Brassylate d'éthylène), propanoate de (2S)-2-propyl-1,1-dimethyl-propoxy ((2S)-Ester propylique de l'acide 2-(1,1-diméthylpropoxy)-propanoïque), acétate de 2-tert-butylcyclohexyle (acétate de 2-(1,1-diméthylethyl)cyclohexyle), salicylate de ci-3-hexenyle, acétate de [(1S)-3-(4-méthylpent-3-ènyl)-1-cyclohex-3-ènyl]méthyle, l'acétate de 3-pentyltetrahydro[2H]pyranyle, propionate de linalyle, acétate de cédryle, acétate d'anisyle, acétate de nopyle, acétate de néryle, acétate de 3a,4,5,6,7,7a-hexahydro-4,7-methanoindèn-6-yle, propanoate de 3a,4,5,6,7,7a-hexahydro-4,7-methano-1H-indèn-6-yl, 3-cyclohexanepropanoate de 2-propényl, 2-hydroxypropane-1,2,3-tricarboxylate de 1,2,3-triéthyle, acétate de (2E)-3,7-diméthylocta-2,6-dièn-1-yle, acétate de 3,5,5-trimethylhexyle, acétate de 3,7-diméthyl-octa-1,6-diene-3-yle, éthanoate de cis-3,7-diméthyl-2,6-octadiènyle, ester de 1-méthyléthyl de l'acide tetradécanoïque, ester 3-méthylbutylique de l'acide 2-hydroxy-benzoïque, ester phénylméthylique de l'acide 2-hydroxy-benzoïque, ester 2-hexylique de l'acide 2-hydroxy-benzoïque, ester méthylique de l'acide 2-hydroxy-Benzoïque, ester éthylique de l'acide acétoacétique, acétate de 3,7-diméthyl-octa-1,6-diène-3-yle, ester 1,2-diéthylique de l'acide 1,2-benzènedicarboxylique, 2-méthylpropanoate de (Z)-hex-3-ènyle, acétate de (4-méthyl-1-propan-2-yl-1-cyclohex-2-enyl), acétate de 3a,4,5,6,7,7a-hexahydro-4,7-méthanoindèn-6-yle, 2,3-époxy-3-phénylbutyrate d'éthyle, 2-aminobenzoate de méthyle, 2-(méthylamino)benzoate de méthyle, benzoate de méthyle, 2,4-dihydroxy-3,6-diméthylbenzoate de méthyle, acétate (3R-(3alpha,3beta,6beta,7beta,8alpha))-octahydro-6-méthoxy-3,6,8,8-tetraméthyl-1H-3a,7-méthanoazulène et le salicylate d'hexyle. De manière préférée, des exemples d'esters comprennent, mais ne sont pas limités à, le propionate de linalyle, acétate de cédryle, acétate d'anisyle, acétate de nopyle, acétate de néryle, acétate (3R-(3alpha,3beta,6beta,7beta,8alpha))-octahydro-6-méthoxy-3,6,8,8-tetraméthyl-1H-3a,7-méthanoazulène et le salicylate d'hexyle. Des exemples d'éthers comprennent, mais ne sont pas limités à, l'éther de benzyle, éther éthylique, ambre gris, oxyde de diphényle, 4,6,6,7,8,8-hexaméthyl-1,3,4,6,7,8-hexahydrocyclopenta[g]isochromene, ambre carane, 1,1-diméthoxy-2,2,5-triméthyl-4-hexène, (éthoxyméthoxy)cyclododecane, (E)-1-méthoxy-4-(1-propényl)-benzène, 1-méthoxy-4-(2-propenyl)-benzène et l'éther éthylique de 2-naphtyle. De manière préférée, des exemples d'éthers comprennent, mais ne sont pas limités à, le 1,1-diméthoxy-2,2,5-triméthyl-4-hexène et l'éther éthylique de 2-naphtyle.

Des exemples d'alcools comprennent, mais ne sont pas limités à, le menthol ([1R-(1alpha,2beta,5alpha)]-5-méthyl-2-isopropylcyclohexanol), citronellol, géraniol, linalol (par exemple l'éthyle linalol et le tetrahydro linalol), alcool phényléthylique, terpinéol, 2,6-diméthylheptan-2-ol, 2-méthyl-1-phénylpropan-2-ol (diméthyl phényle carbinol), 3-méthyl-5-[2,2,3-triméthylcyclopent-3-èn-1-yl]pent-4-èn-2-ol, 2-phényléthanol, 2-éthyl-4-(2,2,3-triméthyl-1-cyclopent-3-enyl)but-2-èn-1-ol, (E)-4-méthyldec-3-èn-5-ol, alcool cinnamique (3-phényl-2-propèn-1-ol), 2,6-diméthyloct-7-èn-2-ol, alpha,beta,2,2,3-pentaméthylcyclopent-3-ène-1-butanol, 3-(5,5,6-triméthylbicyclo[2.2.1]hept-2-yl) cyclohexan-1-ol (IBCH), cis-3-hexèn-1-ol, méthyl-triméthylbicyclo-hexylméthyl-cyclopropyl méthanol alcool benzylique, endo-1,7,7-triméthyl-bicyclo-[2.2.1]heptan-2-ol, 3,7-diméthyl-6-octèn-1-ol, 3,7-diméthyl-1-octanol, (2E)-3,7-diméthyl-2,6-octadièn-1-ol, cis-3,7-diméthyl-2,6-octadièn-1-ol, 3,7-diméthyl-octa-1,6-diène-3-ol, 2-(4-méthyl-1-cyclohex-3-ènyl)propan-2-ol, 4-méthyl-1-(1-méthyléthyl)-3-cyclohexèn-1-ol, (1R, 2S, 5R)-5-méthyl-2-(1-méthyléthyl)-cyclohexanol, (2E)-3,7-diméthyl-2,6-octadièn-1-ol et le 3-méthylbutan-1-ol. De manière préférée, des exemples d'alcools comprennent, mais ne sont pas limités à, l'alpha,beta,2,2,3-pentaméthylcyclopent-3-ène-1-butanol, 3-(5,5,6-triméthylbicyclo[2.2.1]hept-2-yl) cyclohexan-1-ol (IBCH), cis-3-hexenol, méthyl-triméthylbicyclo-hexylméthyl-cyclopropyl méthanol, 3-méthylbutan-1-ol, linalol d'éthyle, tetrahydro linalol et le [lR-(lalpha,2beta,5alpha)]-5-méethyl-2-isopropylcyclohexanol (menthol).

Des exemples d'aldéhydes comprennent, mais ne sont pas limités à, les alcanals linéaires comprenant entre 8 et 18 atomes de carbones, citral, citronellal, aldéhyde de Cyclamen, hydroxycitronellal, 3-(4-tert-butylphényl)-2-methylpropanal, 3-(4-tert-butylphényl)propanal, 2,6,10-triméthylundec-9-ènal, 4(octahydro-4,7-méthano[5H] indèn-5-ylidène)butanal, 3-(3-propan-2-ylphényl)butanal, 7-hydroxy-3,7-diméthyloctanal (hydroxycitronellal, 3,7-diméthyl-7-hydroxy-octane-1-al), 4-(4-hydroxy-4-méthylpentyl)cyclohex-3-ène-1-carbaldehyde, octahydro-5-méthoxy-4,7-méthano-1H-indène-2-carboxaldehyde, aldéhyde d'alpha-méthyle cinnamique (2-méthyl-3-phényl-2-propènal), 4-méthoxybenzaldehyde (Aldéhyde anisique), aldéhyde en C10 (décanal), undéc-10-enal, aldéhyde en C12 (laurique ou dodécanal), acétaldéhyde de méthyl-nonyle (2-méthylundécanal), aldéhyde en C16, aldéhyde en C6 (hexanal), aldéhyde cinnamique (3-phényl-2-propénal), 3-éthyoxy-4-hydroxybenzaldéhyde (Ethylvanilline), aldéhyde d'hexyl cinnamique (2-benzylidèneheptanal), 3-phénylbutanal (3-phénylbutyraldehyde), 2,4-diméthylcyclohex-3-ène-1-carbaldehyde, 5-heptanal, 2,6-dimethylhept-5-enal, 4-hydroxy-3-méthoxybenzaldehyde (Vanilline), alpha-méthyl-1,3-benzodioxole-5-propionaldehyde, 4-isopropylbenzaldehyde, 3,7-dimethyl-6-octènal, 3,7-diméthyl-2,6-octadiènal, 4-(4-hydroxy-4-méthylpentyl)-3-cyclohexène-1-carboxaldehyde, trans-hex-2-ènal, 2,4,6-triméthyl-3-cyclohexène-1-carboxaldehyde, 2-(4-tert-butylbenzyl)propionaldehyde et le benzaldehyde. De manière préférée, des exemples d'aldéhydes comprennent, mais ne sont pas limités à, le 2,4-diméthylcyclohex-3-ène-1-carbaldehyde, 5-heptanal, 2,6-diméthyl-hept-5-enal, 4-hydroxy-3-méthoxybenzaldehyde (Vanilline), alpha-méthyl-1,3-benzodioxole-5-propionaldehyde et le benzaldehyde.

Des exemples de cétones comprennent, mais ne sont pas limités à, les ionones, isométhylionone, cédryle de méthyle, (E)-1-(2,6,6-triméthyl-1-cyclohex-2-enyl)but-2-en-1-one (alpha-damascone), 3-méthyl-2-[(2Z)-pent-2-en-1-yl]cyclopent-2-en-1-one (cis-jasmone), 4-(4-méthoxyphényl)-butan-2-one, 4(3)-(4-méthylpent-3-ènyl)cyclohex-3-ènecarbaldehyde, cétone de méthyl cédryl, 7-méthylbenzo[b][1,4]dioxepin-3-one, 1,7,7-triméthylbicyclo[2,2,1]heptan-2-one, 1-benzopyrane-2-one (Coumarine), 1-(2,6,6-triméthyl-1-cyclohex-3-ènyl)but-2-èn-1-one, butan-2,3-dione (Diacétyle), 1-(1,2,3,4,5,6,7,8-octahydro-2,3,8,8,-tetraméthyl-2-naphthyl)éthan-1-one, irones, 1-(2-naphthalényl)éthanone (2-acétonaphtone), menthone, carvone, 3-méthyl-2-pentyl-2-cyclopentènone, 1-(2,6,6-triméthyl-3-cyclohexen-1-yl)-2-butèn-1 -one, 1-(2,6,6-triméthyl-2-cyclohexényl)hepta-1,6-dièn-3-one, 2-éthyl-3-hydroxy-4H-pyran-4-one, (5R)-2-méthyl-5-prop-1-èn-2-ylcyclohex-2-èn-1-one, 1-(6-tert-butyl-1,1-diméthyl-2,3-dihydro-1H-indèn-4-yl)éthanone, 1-(5,6,7,8-tetrahydro-3,5,5,6,8,8-hexaméthyl-2-naphthyl)éthan-1-one, 4-(2,6,6-triméthylcyclohex-2-ènyl)-but-3-ène-2-one, octan-2-one et la 1,3,4,6,7,8a-Hexahydro-1,1,5,5-tetramethyl-2H-2,4a-methanonaphthalen-8(5H)-one. De manière préférée, des exemples de cétones comprennent, mais ne sont pas limités à, les irones, 1-(2-naphthalényl)éthanone (2-acétonaphtone), menthone, carvone, 3-méthyl-2-pentyl-2-cyclopentènoneet la 1,3,4,6,7,8a-Hexahydro-1,1,5,5-tetramethyl-2H-2,4a-methanonaphthalen-8(5H)-one.

Des exemples de lactones comprennent, mais ne sont pas limités à, la gamma décalactone (decan-4-olide), gamma undécalactone (undecan-4-olide), cis-jasmone lactone, gamma undecalactone, delta octalacone, delta decalactone et la hexahydro-3,6-diméthyl-2(3H)-benzofuranone. De manière préférée, des exemples de lactones comprennent, mais ne sont pas limités à, la gamma undecalactone, delta octalacone, delta decalactone et la hexahydro-3,6-diméthyl-2(3H)-benzofuranone.

Des exemples d'acétals comprennent, mais ne sont pas limités à, le 2,4- diméthyl tetrahydroindenodioxine, diacétal de l'aldéhyde phénylacétique, le glycérylacétal de phénylacetaldéhyde, le diéthyl acétal de citral, le diméthyl acétal de citral, 2,6-octadiènal, 1,1-diméthoxy-2-phényléthane et l'acide 3,7-diméthyl isomérisé. De manière préférée, des exemples d'acétals comprennent, mais ne sont pas limités à, le glycérylacétal de phénylacetaldéhyde, le diéthyl acétal de citral, le diméthyl acétal de citral, 2,6-octadiènal et l'acide 3,7-diméthyl isomérisé.

Des exemples de nitriles comprennent, mais ne sont pas limités à, le 3,7-diméthyloct-6-ène nitrile (citronellyle nitrile), tridec-2-ènenitrile, 3-phényl-2-propenènitrile, dodécanenitrile et le 3,7-diméthylnona-2,6-dienènitrile. De manière préférée, des exemples de nitriles comprennent, mais ne sont pas limités à, le tridec-2-ènenitrile, 3-phényl-2-propenènitrile, dodécanenitrile et le 3,7-diméthylnona-2,6-dienènitrile.

Des exemples de phénols comprennent, mais ne sont pas limités à, l'eugénol (2-méthoxy-4-(2-propényl)-phénol), iso-eugénol, 5-méthyl-2-(1-méthyléthyl)-phénol, 2-éthoxy-4-méthylphénol, 2,6-di-tert-butyl-p-crésol et le 2-éthoxy-4-(méthoxyméthyl)-phénol.

Des exemples d'acides comprennent, mais ne sont pas limités à, l'acide pentanoïque, l'acide butyrique et l'acide 2-méthylpent-2-èn-1-oïque.

Des exemples de terpènes tels que des hydrocarbures terpéniques cycliques (par exemple sesquiterpéniques) ou non cycliques, comprennent, mais ne sont pas limités à, le limonène, 1-méthyl-4-isopropényl-1-cyclohexène, 1-méthyl-4-isopropyl-1,4-cyclohexadiène, 7-méthyl-3-méthylèneocta-1,6-diène, 1-méthyl-4-(1-méthyléthyl)-1,3-cyclohexadiène, 2,6,6-triméthylbicyclo[3.1.1]hept-2-ène, 6,6-diméthyl-2-méthylènebicyclo[3.1.1]heptane, 2,2-diméthyl-3-méthylènebicyclo-[2.2.1]-heptane, [1R,(1R*,4E,9S*)]-4,11,11-triméthyl-8-méthylène-bicyclo[7.2.0]undec-4-ène, 1-méthyl-4-(1-méthyléthyl)benzène et les huiles essentielles à sesquiterpènes. De manière préférée, des exemples de terpènes comprennent, mais ne sont pas limités à, des huiles essentielles à sesquiterpènes.

Des exemples de composés hétérocycliques azotés ou soufrés, saturés ou insaturés, comprennent, mais ne sont pas limités à, l'indole, 1,3-benzopyrrole, tétrahydro-4-méthyl-2-(2-méthyl-1-propényl)-2H-pyrane, 2-méthyl-pyrazine, 4-méthyl-5-hydroxyéthyl thiazole (2-(4-méthylthiazol-5-yl)éthanol), 6-tert-butylquinoline, 6-(isopropyl)quinoline, cis-2-méthyl-4-propyl-1,3-oxathianeet les pyrazines. De manière préférée, des exemples de composés hétérocycliques azotés ou soufrés, saturés ou insaturés, comprennent, mais ne sont pas limités à, la 6-tert-butylquinoline, 6-(isopropyl)quinoline, cis-2-méthyl-4-propyl-1,3-oxathiane et les pyrazines.

Des exemples de produits d'origine naturelle comprennent, mais ne sont pas limités à, des huiles essentielles extraites à partir des différentes parties de plante (fleurs, tiges, feuilles, fruits, écorces, racines, parties boisées, herbes, aiguilles, sève et gommes), des résinoïdes, des concrètes, ou absolues obtenues à partir de ces derniers. De manière préférée, des exemples de produits d'origine naturelle comprennent, mais ne sont pas limités à, de l'huile d'armoise herbe blanche *(Artemisia Herba-Alba*), extrait de l'écorce de citronnier (Citrus Limon Peel extract), de l'huile de feuille *d'Eucalyptus globulus,* huile de *Dipterocarpus turbinatus (Dipterocarpus turbinatus Balsam Oil),* huile de feuille de *Pogostemon cablin,* huile de feuille de *Rosmarinus officinalis,* huile de *Juniperus virginiana,* huile de feuille de menthe des champs *(Mentha Arvensis),* huile de feuille de menthe verte *(Mentha viridis),* huile d'écorce de *Citrus Aurantium Dulcis,* extrait d'écorce de *Citrus Aurantium Dulcis,* huile de feuille de Cyprès méditerranéen *(Cupressus sempervirens*), *Huile de feuille de patchouli (Pogostemon cablin),* huile de Cèdre Texas *(Juniperus Mexicana*) et l'huile de Lavandula Hybride *(Lavandula Hybrida).*

La composition parfumée peut comprendre des ingrédients d'origine naturelle ou synthétique. Le choix de cette composition parfumée dépend d'une part de l'effet odorant recherché et d'autre part de la nature du produit qui la contient.

La composition parfumée selon l'invention est destinée à être appliquée ou vaporisée sur une surface ou dans l'air afin de conférer une odeur agréable.

Par « odeur agréable », on entend une odeur qui est détectée par le sens olfactif de l'être humain et qui est perçue comme agréable.

La composition parfumée selon l'invention peut en outre comprendre des agents bactéricides, bactériostatiques, insecticides et agents répulsifs.

La présente invention a également pour objet une base cosmétique comprenant le mélange ou la composition parfumée de la présente invention.

Par « base cosmétique », on entend un produit cosmétique ou un produit de soin d'hygiène sous forme d'une crème, d'une émulsion, d'une mousse, d'une cire, d'une huile, d'une lotion, d'un gel, d'une suspension, d'une solution, d'une poudre, d'un baume, d'un sérum, d'un masque ou d'un gommage. Des exemples de produits cosmétiques, comprennent, mais ne sont pas limités à, des parfums, eaux de parfums, eaux de toilette, produits capillaires, produits de rasage et d'après-rasage, huiles essentielles, soins pour la peau, déodorants, anti-transpirants, produits dépilatoires, produits auto-bronzants, protections solaires, produits de maquillage, etc. Des exemples de soins d'hygiène comprennent, mais ne sont pas limités à, des lingettes, talcs, couches, bavoirs, mouchoirs, serviettes en papiers, savons, gel douches, shampooings et autres produits de nettoyage corporel, soins bucco-dentaires, produits d'hygiène féminine, etc.

De tels produits peuvent être aussi bien destinés aux êtres humains qu'aux animaux.

La présente invention a également pour objet une base détergente comprenant le mélange ou la composition parfumée de la présente invention.

Par « base détergente », on entend des produits d'entretien ou de nettoyage. Des exemples de produits d'entretien ou de nettoyage comprennent, mais ne sont pas limités à, des détergents pour surfaces, pour textiles et pour vaisselle, des agents de blanchiment, agents adoucissants, agents assouplissants, produits décapants, vernis et autres produits ménagers.

La présente invention a également pour objet des produits désherbants et engrais comprenant le mélange ou la composition parfumée de la présente invention.

La présente invention a également pour objet un parfum d'ambiance comprenant le mélange ou la composition parfumée de la présente invention.

Par « parfum d'ambiance », on entend des produits destinés à parfumer l'air. Les produits d'ambiance peuvent, de façon non limitative, être contenus dans des aérosols, vaporisateurs, sprays, bougies, gels parfumés, supports solides, brûles parfums, encens, billes et diffuseurs de parfums. Le parfum d'ambiance de la présente invention peut être utilisé pour les espaces clos publics, professionnels ou privés; par exemple, la voiture, la maison, les bâtiments administratifs, les transports en commun, les musées, les monuments historiques, les églises, les caves, les écoles, les restaurants, les cinémas, les hôpitaux, les usines, les stations de traitement d'eau ou de déchets, les boutiques et les hôtels. Le parfum d'ambiance peut également être utilisé dans les systèmes à air conditionné.

La présente invention concerne également des matériaux comprenant ou recouverts par le mélange ou par la composition parfumée selon l'invention, notamment des polymères comme par exemple des plastiques, des macromolécules comme par exemple la cellulose, etc.

Le mélange ou la composition parfumée selon l'invention sont destinés à être appliqués sous forme concentrée ou non dans des bases cosmétiques, bases détergentes ou parfums d'ambiance.

La présente invention a également pour objet l'utilisation du mélange selon l'invention pour préparer une composition cosmétique, une base détergente ou un parfum d'ambiance.

La présente invention a également pour objet l'utilisation du mélange, de la composition parfumée selon l'invention, de la base cosmétique, de la base détergente ou du parfum d'ambiance pour masquer les mauvaises odeurs.

Par « mauvaises odeurs », on entend des odeurs qui sont détectées par le sens olfactif de l'être humain et qui sont perçues comme offensantes ou désagréables. Au sens de la présente invention, les mauvaises odeurs à masquer peuvent résulter de manière intrinsèque de la base cosmétique, détergente ou du parfum d'ambiance lui-même, ou bien provenir de l'environnement extérieur.

Par « masquer les mauvaises odeurs » on entend la modification de la perception qualitative de l'odeur via l'ajout d'une substance ou d'un mélange de substance capable de fournir une odeur nouvelle, distincte et agréable.

Dans un mode de réalisation particulier, le mélange ou la composition parfumée de la présente invention masquent les mauvaises odeurs sans pour autant dégrader les molécules responsables des mauvaises odeurs. En effet, les molécules du mélange masquant de la composition parfumée masquent olfactivement les molécules responsables des mauvaises odeurs.

La présente invention sera décrite plus en détail à l'aide des exemples suivants qui ont un caractère purement illustratif.

### EXEMPLES

### Exemple 1 : Composition parfumée No. 1

Le mélange masquant et les autres composants de la composition parfumée No. 1, décrits dans le tableau 1, sont mélangés afin d'obtenir la composition parfumée.

**Tableau 1**

| **Désignation du produit** | **% En poids Quantité** |
|---|---|
| Ester 2-propénylique de l'acide 3-méthylbutoxy-acétique | 0,027 |
| Acétate d'isopentyle | 0,667 |
| Acétate de (Z)-hex-3-ènyle | 0,100 |
| Acétate de 3,7-diméthyl-6-octèn-1-ol | 0,033 |
| Acétate d'hexyle | 0,667 |
| Exo-acétate de bicyclo[2.2.1]heptan-2-ol,1,7,7-triméthyle | 0,333 |
| Acétate d'alpha,alpha,4-triméthylcyclohexylméthyle | 0,533 |
| Acétate de 3-méthyl-2-butényle | 0,167 |
| Acétate de 4-méthyl-1-propan-2-yl-1-cyclohex-2-ènyle | 0,667 |
| 3-phényl-2-propèn-1-ol | 4,000 |
| 2-méthyl-3-phényl-2-propènal | 4,667 |
| p- méthoxybenzaldehyde | 1,333 |
| Décanal | 0,067 |
| Dodécanal | 0,047 |
| 2-méthylundécanal | 0,067 |
| 2,3-époxy-3-phénylbutyrate d'éthyle | 0,101 |
| Dihydro-5-pentyl-2(3H)-furanone | 4,667 |
| Hexanal | 0,027 |
| 3-phényl-2-propénal | 0,533 |
| 2-phényléthanol | 0,133 |
| Formiate d'alpha,3,3-triméthylcyclohexylméthyle | 0,500 |
| Butanoate de 3-méthylbutyle | 0,167 |
| 1,7,7-triméthylbicyclo[2.2.1]-2-heptanone | 0,133 |
| 3,7-diméthyloct-6-ènenitrile | 0,007 |
| 2H-1-benzopyran-2-one | 1,000 |
| Acétate de 3a,4,5,6,7,7a-hexahydro-4,7-methanoindèn-6-yle | 0,333 |
| Propanoate de 3a,4,5,6,7,7a-hexahydro-4,7-methano-1H-indèn-6-yl | 0,333 |
| 3-cyclohexanepropanoate de 2-propényl | 0,120 |
| 1-(2,6,6-triméthyl-3-cyclohexen-1-yl)-2-butèn-1-one | 0,133 |
| 2,6-diméthyloct-7-èn-2-ol | 1,333 |
| 2,3-butanedione | 0,007 |
| 1,1'-oxydipropan-2-ol | 47,527 |
| 3-éthoxy-4-hydroxybenzaldéhyde | 1,333 |
| 2-méthoxy-4-(2-propényl)-phénol | 0,667 |
| Undecan-4-olide | 0,002 |
| Butanoate d'alpha,alpha-diméthylphenethyl | 0,073 |
| 1-(2,6,6-triméthyl-2-cyclohexényl)hepta-1,6-dièn-3-one | 0,003 |
| 2-methylpropanoate de 2-phénoxyéthyle | 6,720 |
| 2-éthyl-3-hydroxy-4H-pyran-4-one | 0,667 |
| 2-hydroxypropane-1,2,3-tricarboxylate de 1,2,3-triéthyle | 0,200 |
| 2-méthyl-pyrazine | <10 ppm |
| Decan-4-olide | 1,333 |
| Heptanoate de 2-propényl | 0,333 |
| 1-(1,2,3,4,5,6,7,8-octahydro-2,3,5,5-tétraméthyl-2-naphthyl)éthan-1-one | 0,200 |
| 2-benzylidèneheptanal | 3,000 |
| 2,4-diméthylcyclohex-3-ène-1-carbaldehyde | 0,800 |
| 2-méthylpentanoate d'éthyle | 0,067 |
| Ester éthylique de l'acide 2-méthyl-butanoïque | 1,333 |
| 1,4-dioxacycloheptadecane-5,17-dione | 1,667 |
| (2S)-Ester propylique de l'acide 2-(1,1-diméthylpropoxy)-propanoïque | 0,133 |
| 2-(4-méthylthiazol-5-yl)éthanol | 0,027 |
| 3 -phénylbutyraldehyde | 0,007 |
| 4-méthyldec-3-èn-5-ol | 0,007 |
| Acétate de 2-(1,1-diméthylethyl)cyclohexyle | 10,000 |
| **2,4-diméthyl-4-phényltetrahydrofurane** | **0,350** |
| **ester éthylique de l'acide 10-undécénoïque** | **0,300** |
| **Dihydro-5-pentyl-2(3H)-furanone** | **0,350** |

### Exemple 2 : Composition parfumée No. 2

Le mélange masquant et les autres composants de la composition parfumée No. 2, décrits dans le tableau 2, sont mélangés afin d'obtenir la composition parfumée.

**Tableau 2**

| **Désignation du produit** | **% En poids Quantité** |
|---|---|
| Ester 2-propénylique de l'acide 3-méthylbutoxy-acétique | 0,444 |
| ester phénylméthylique de l'acide acétique | 0,889 |
| Acétate de (3R-(3alpha,3abcta,6alpha, 7beta,8aalpha))-octahydro-3,6,8,8-tetraméthyl-1H-3a,7-méthanoazulèn-5-yle | 0,370 |
| Acétate de (2E)-3,7-diméthylocta-2,6-dièn-1-yle | 0,013 |
| Exo-acétate de bicyclo[2.2.1]heptan-2-ol,1,7,7-triméthyle | 0,461 |
| Acétate de 3,5,5-trimethylhexyle | 0,111 |
| Acétate de 3,7-diméthyl-octa-1,6-diene-3-yle | 5,926 |
| Ethanoate de cis-3,7-diméthyl-2,6-octadiènyle | 0,017 |
| Acétate de 4-méthyl-1-propan-2-yl-1-cyclohex-2-ènyle | 0,370 |
| Undéc-10-enal | 0,037 |
| Undécan-4-olide | 0,044 |
| 4-isopropylbenzaldehyde | 0,052 |
| 2,6,6-triméthylbicyclo[3.1.1]hept-2-ène | 0,236 |
| 2-phényléthanol | 0,148 |
| Huile d'armoise herbe blanche *(Artemisia Herba-Alba)* | 0,296 |
| Benzoate de méthyle | 0,015 |
| 6,6-diméthyl-2-méthylènebicyclo[3.1.1]heptane | 1,065 |
| 2,6-di-tert-butyl-p-crésol | 0,185 |
| (Ethoxyméthoxy)cyclododecane | 0,074 |
| Endo-1,7,7-triméthyl-bicyclo-[2.2.1]heptan-2-ol | 0,204 |
| 7-méthyl-2H-benzo-1,5-dioxepin-3(4H)-one | 0,007 |
| 2,2-diméthyl-3-méthylènebicyclo-[2.2.1]-heptane | 0,213 |
| 1,7,7-triméthylbicyclo[2.2.1]-2-heptanone | 0,702 |
| (5R)-2-méthyl-5-prop-1-èn-2-ylcyclohex-2-èn-1-one | 0,185 |
| [1R,(1R*,4E,9S*)]-4,11,11-triméthyl-8-méthylène-bicyclo[7.2.0]undec-4-ène | 0,320 |
| extrait de l'écorce de citronnier | 0,244 |
| (E)-1-méthoxy-4-(1-propényl)-benzène | 0,370 |
| 3,7-dimethyl-6-octènal | 0,002 |
| 3,7-diméthyl-6-octèn-1-ol | 0,006 |
| 2H-1-benzopyran-2-one | 0,407 |
| Huile de feuille de Cyprès méditerranéen (*Cupressus sempervirens*) | 0,148 |
| 2,6-diméthyloct-7-èn-2-ol | 4,815 |
| 3,7-diméthyl-1-octanol | <10 ppm |
| 1-méthyl-4-isopropényl-1-cyclohexène | 0,019 |
| 1,1'-oxydipropan-2-ol | 52,837 |
| 1-méthoxy-4-(2-propenyl)-benzène | 0,222 |
| Huile de feuille d'*Eucalyptus globulus* | 1,956 |
| 2-méthoxy-4-(2-propényl)-phénol | 0,519 |
| 4,6,6,7,8,8-hexaméthyl-1,3,4,6,7,8-hexahydrocyclopenta[g] isochromène | 0,037 |
| 1-méthyl-4-isopropyl-1,4-cyclohexadiène | 0,036 |
| Ester de 1-méthyléthyl de l'acide tetradécanoïque | 0,036 |
| (2E)-3,7-diméthyl-2,6-octadièn-1-ol | <10 ppm |
| Huile de Dipterocarpus turbinatus *(Dipterocarpus turbinatus balsam oil)* | 0,074 |
| 3,7-diméthyl-7-hydroxy-octane-1-al | 0,519 |
| 6-tert-butylquinoline | 0,089 |
| huile de Lavandula Hybride *(Lavandula Hybrida*) | 0,296 |
| 3,7-diméthyl-2,6-octadiènal | 0,137 |
| 2,4-diméthylcyclohex-3-ène-1-carbaldehyde | 0,007 |
| 3,7-diméthyl-octa-1,6-diène-3-ol | 5,613 |
| 4-(4-hydroxy-4-méthylpentyl)-3-cyclohexène-1-carboxaldehyde | 0,296 |
| 2,4-dihydroxy-3,6-diméthylbenzoate de méthyle | 0,296 |
| 7-méthyl-3-méthylèneocta-1,6-diène | 0,107 |
| cis-3,7-diméthyl-2,6-octadièn-1-ol | 0,016 |
| Huile d'écorce de *Citrus Aurantium Dulcis* | 1,852 |
| Extrait d'écorce de *Citrus Aurantium Dulcis* | 2,378 |
| Tétrahydro-4-méthyl-2-(2-méthyl-1-propényl)-2H-pyrane | 0,296 |
| 1-méthyl-4-(1-méthyléthyl)benzène | 0,071 |
| *Huile de feuille de patchouli (Pogostemon cablin)* | 1,111 |
| Huile de feuille de *Rosmarinus officinalis* | 0,044 |
| Ester 3-méthylbutylique de l'acide 2-hydroxy-benzoïque | 0,741 |
| Ester phénylméthylique de l'acide 2-hydroxy-benzoïque | 10,741 |
| Ester 2-hexylique de l'acide 2-hydroxy-benzoïque | 0,022 |
| 2-(4-méthyl-1-cyclohex-3-ènyl)propan-2-ol | 0,072 |
| 4-méthyl-1-(1-méthyléthyl)-3-cyclohexèn-1-ol | 0,004 |
| 1-méthyl-4-(1-méthyléthyl)-1,3-cyclohexadiène | 0,150 |
| 5-méthyl-2-(1-méthyléthyl)-phénol | 0,027 |
| **2,4-diméthyl-4-phényltetrahydrofurane** | **0,500** |
| **Dihydro-5-pentyl-2(3H)-furanone** | **0,500** |

### Exemple 3 : Composition parfumée No.3

Le mélange masquant et les autres composants de la composition parfumée No. 3, décrits dans le tableau 3, sont mélangés afin d'obtenir la composition parfumée.

**Tableau 3**

| **Désignation du produit** | **% En poids Quantité** |
|---|---|
| Exo-acétate de 1,7,7-triméthyl-bicyclo[2.2.1]heptan-2-ol | 6,667 |
| (5R)-2-méthyl-5-prop-1-èn-2-ylcyclohex-2-èn-1-one | 0,083 |
| Huile de *Juniperus virginiana* | 0,083 |
| 1,1'-oxydipropan-2-ol | 79,508 |
| Huile de feuille d'*Eucalyptus globulus* | 6,667 |
| 2,4-diméthylcyclohex-3-ène-1-carbaldehyde | 0,025 |
| Huile de feuille de menthe des champs *(Mentha Arvensis)* | 1,667 |
| *Huile de feuille de menthe verte (Mentha viridis)* | 3,333 |
| 2-(4-méthyl-1-cyclohex-3-ènyl)propan-2-ol | 0,917 |
| Ester méthylique de l'acide 2-hydroxy-Benzoïque | 0,050 |
| **2,4-diméthyl-4-phényltetrahydrofurane** | **0,250** |
| **Dihydro-5-pentyl-2(3H)-furanone** | **0,750** |

### Exemple 4 : Composition parfumée No. 4

Le mélange masquant et les autres composants de la composition parfumée No. 4, décrits dans le tableau 4, sont mélangés afin d'obtenir la composition parfumée.

**Tableau 4**

| **Désignation du produit** | **% En poids Quantité** |
|---|---|
| Ester éthylique de l'acide acétoacétique | 1,500 |
| Acétate d'isopentyle | 1,500 |
| Acétate de (Z)-hex-3-ènyle | 0,100 |
| Dihydro-5-pentyl-2(3H)-furanone | 0,100 |
| Acétate de 1,1-diméthyl-2-phényléthyle | 1,500 |
| Acétate de 3,7-diméthyl-octa-1,6-diène-3-yle | 0,100 |
| Acétate de 1-phényléthyle | 0,600 |
| Alcool benzylique | 0,500 |
| Undécan-4-olide | 3,500 |
| cis-hex-3-èn-1-ol ; cis-3-hexenol | 0,150 |
| 1-(6-tert-butyl-1,1-diméthyl-2,3-dihydro-1H-indèn-4-yl)éthanone | 0,100 |
| 3-cyclohexanepropanoate de 2-propényl | 0,200 |
| 2,6-diméthyloct-7-èn-2-ol | 1,000 |
| 2-méthyl-1-phénylpropan-2-ol | 0,400 |
| 1,1'-oxydipropan-2-ol | 59,780 |
| 1-(5,6,7,8-tetrahydro-3,5,5,6,8,8-hexaméthyl-2-naphthyl)éthan-1-one | 4,500 |
| 4,6,6,7,8,8-hexaméthyl-1,3,4,6,7,8-hexahydrocyclopenta[g] isochromène | 1,000 |
| Ester 1,2-diéthylique de l'acide 1,2-benzènedicarboxylique | 1,000 |
| Heptanoate de 2-propényl | 2,000 |
| Trans-hex-2-ènal | 0,150 |
| 4-(2,6,6-triméthylcyclohex-2-ènyl)-but-3-ène-2-one | 0,300 |
| 2,4,6-triméthyl-3-cyclohexène-1-carboxaldehyde | 0,600 |
| 2-méthylpropanoate de (Z)-hex-3-ènyle | 0,100 |
| 2-méthylpropanoate de 2-phénoxyéthyle | 5,500 |
| 2-benzylidèneheptanal | 0,500 |
| 3,7-diméthyl-2,6-octadiènal | 0,100 |
| 2,4-diméthylcyclohex-3-ène-1-carbaldehyde | 0,400 |
| Ester éthylique de l'acide 2-methyl-butanoïque | 0,500 |
| 1,4-dioxacycloheptadecane-5,17-dione | 0,100 |
| Extrait d'écorce de *Citrus Aurantium Dulcis* | *0,100* |
| 2-éthyl-3-hydroxy-4H-pyran-4-one | 0,020 |
| Acétate de 2-(1,1-diméthyléthyl)cyclohexyle | 11,000 |
| Acétate de 4-tert-butylcyclohexyle | 0,100 |
| **2,4-diméthyl-4-phényltetrahydrofurane** | **0,350** |
| **(3E)-4-(2,6,6-triméthylcyclohex-2-èn-1-yl)but-3-èn-2-one** | **0,300** |
| **Dihydro-5-pentyl-2(3H)-furanone** | **0,350** |

### Exemple 5 : Composition parfumée No. 5

Le mélange masquant et les autres composants de la composition parfumée No. 5, décrits dans le tableau 5, sont mélangés afin d'obtenir la composition parfumée.

**Tableau 5**

| **Désignation du produit** | **% En poids Quantité** |
|---|---|
| Ester 2-propénylique de l'acide (3-methylbutoxy)-acétique | 0,688 |
| Acétate de 3,7-diméthyl-6-octèn-1-ol | 0,500 |
| Acétate de (2E)-3,7-diméthylocta-2,6-dièn-1-yle | 0,063 |
| Acétate d'hexyle | 1,563 |
| Exo-acétate de bicyclo[2.2.1]heptan-2-ol-1,7,7-triméthyle | 5,000 |
| Acétate de 3,7-diméthyl-octa-1,6-diène-3-yle | 3,125 |
| Acétate d'alpha,alpha,4-triméthylcyclohexylméthyle | 4,375 |
| Acétate de (4-méthyl-1-propan-2-yl-1-cyclohex-2-enyl) | 1,375 |
| Hexan-1-ol | 0,563 |
| Dodécanal | 0,188 |
| Endo-1,7,7-triméthyl-bicyclo-[2.2.1]heptan-2-ol | 0,375 |
| 1,7,7-triméthylbicyclo[2.2.1]-2-heptanone | 2,000 |
| [1R,(1R*,4E,9S*)]-4,11,11-triméthyl-8-méthylène-bicyclo[7.2.0]undec-4-ène | 0,125 |
| Huile de Cèdre de Texas *(Juniperus Mexicana)* | 6,250 |
| 2H-1-benzopyran-2-one | 1,625 |
| Acétate de 3a,4,5,6,7,7a-hexahydro-4,7-méthanoindèn-6-yle | 4,000 |
| 2,6-diméthyloct-7-èn-2-ol | 5,250 |
| 1,1'-oxydipropan-2-ol | 43,938 |
| Huile de feuille d'*Eucalyptus globulus* | 2,500 |
| huile de Lavandula Hybride *(Lavandula Hybrida)* | 1,250 |
| 3,7-diméthyl-octa-1,6-diène-3-ol | 3,750 |
| Huile de feuille de menthe verte *(Mentha viridis)* | 1,250 |
| (1R, 2S, 5R)-5-méthyl-2-(1-méthyléthyl)-cyclohexanol | 3,750 |
| Octan-2-one | 0,875 |
| Huile de feuille de *Rosmarinus officinalis* | 0,625 |
| Acétate de 4-tert-butylcyclohexyle | 4,000 |
| **2,4-diméthyl-4-phényltetrahydrofurane** | **0,200** |
| **(3E)-4-(2,6,6-triméthylcyclohex-2-èn-1-yl)but-3-èn-2-one** | **0,200** |
| **Dihydro-5-pentyl-2(3H)-furanone** | **0,600** |

### Exemple 6 : Composition parfumée No. 6

Le mélange masquant et les autres composants de la composition parfumée No. 6, décrits dans le tableau 6, sont mélangés afin d'obtenir la composition parfumée.

**Tableau 6**

| **Désignation du produit** | **% En poids Quantité** |
|---|---|
| Ester phénylméthylique de l'acide acétique | 20,000 |
| Acétate de (Z)-Hex-3-ènyl | 0,200 |
| Acétate de 1-phényléthyle | 0,600 |
| cis-hex-3-èn-1-ol; cis-3-hexenol | 0,200 |
| 3,7-diméthyl-6-octèn-1-ol | 2,608 |
| 3,7-diméthyl-1-octanol | 0,140 |
| 1,1'-oxydipropan-2-ol | 25,100 |
| (2E)-3,7-diméthyl-2,6-octadièn-1-ol | 0,123 |
| 1,3-benzopyrrole | 0,500 |
| 2-benzylidèneheptanal | 10,000 |
| 2,4-diméthylcyclohex-3-ène-1-carbaldehyde | 0,400 |
| 2-(4-tert-butylbenzyl)propionaldehyde | 26,000 |
| 3,7-diméthyl-octa-1,6-diène-3-ol | 10,000 |
| cis-3,7-diméthyl-2,6-octadièn-1-ol | 0,630 |
| 1,1-diméthoxy-2-phényléthane | 2,500 |
| **2,4-diméthyl-4-phényltetrahydrofurane** | **0,300** |
| **ester éthylique de l'acide 10-undécénoïque** | **0,200** |
| **(3E)-4-(2,6,6-triméthylcyclohex-2-èn-1-yl)but-3-èn-2-one** | **0,200** |
| **Dihydro-5-pentyl-2(3H)-furanone** | **0,300** |

### Exemple 7 : Test de réduction de mauvaises odeurs

Les compositions parfumées sont testées en analyse sensorielle afin de prouver leur efficacité de masquage contre les mauvaises odeurs. Les méthodes utilisées sont issues de normes internationales, applicables dans le domaine des industries, et faisant appel à des analyses olfactives :
- ISO 5492 (Analyse sensorielle - vocabulaire),
- ISO 6658 : 2005 (Analyse sensorielle - Méthodologie - Lignes directrices générales)
- NF V 09-006 (Analyse sensorielle - Méthodologie - Initiation et entraînement à la détection et à la reconnaissance des odeurs),
- ASTM E1958-12 (Standard Guide for Sensory claim substantiation),
- ASTM E1593-13 (Standard Guide for Assessing the efficacy of Air Care products in reducing the perception in indoor malodor).

Ces analyses sensorielles permettent de définir les propriétés masquantes, de dresser un profil sensoriel et de discriminer les compositions parfumées.

### Préparation du test

Trois mauvaises odeurs sont préparées : toilettes, cuisine et tabac. Ainsi, trois tests distincts sont réalisés pour chacune des mauvaises odeurs.

### Mauvaise odeur de toilettes :

Les composés qui sont décrits dans le tableau 7 sont mélangés puis mis en solution à 10% dans l'alcool afin d'obtenir une mauvaise odeur de toilettes.

**Tableau 7**

| **Désignation du produit** | **% en poids** |
|---|---|
| Scatole | 2,7% |
| B-Thionaphtol | 1 % |
| Acide thioglycolique | 21 % |
| Acide caproïque | 6 % |
| Acétate de p-cresyl phényle | 1 % |
| Acide isovalérianique | 2 % |
| N-méthyle morpholine | 6 % |
| Dipropylène glycol | 60,3% |

### Mauvaise odeur de cuisine :

Les composés qui sont décrits dans le tableau 8 sont mélangés puis mis en solution à 10% dans l'alcool afin d'obtenir une mauvaise odeur de cuisine.

**Tableau 8**

| **Désignation du produit** | **% en poids** |
|---|---|
| Triméthylamine | 0,1 % |
| Sulfure de méthyle | 0,13 % |
| Diméthyldisulfure | 0,02 % |
| 2-acétyl pyridine | 3,5 % |
| Acide isovalérianique | 0,7 % |
| Acide butyrique | 0,3 % |
| Acide caprique | 0,5 % |
| Méthyl amyle cétone | 0,1 % |
| Algue Absolue | 2 % |
| 2-mercapto propanone | 0,06 % |
| Butyrolactate de butyle | 1,3 % |
| Methional | 0,02 % |
| Indole (1% Dipropylène glycol) | 0,14 % |
| Diacétyle | 3,5 % |
| Acide caproïque | 0,2 % |
| Guaïacol | 0,04 % |
| Base huître | 0,2 % |
| Base saumon | 0,2 % |
| Base crevette | 0,2 % |
| Dipropylène glycol | 78,79 % |
| Sulfate d'allyle | 6 % |
| Mélange d'huile d'ail | 2 % |

### Mauvaise odeur de tabac :

Les composés qui sont décrits dans le tableau 9 sont mélangés puis mis en solution à 10% dans l'alcool afin d'obtenir une mauvaise odeur de tabac.

**Tableau 9**

| **Désignation du produit** | **% en poids** |
|---|---|
| Furfural | 5 |
| Pyridine | 0,1 |
| Créosote de bois de hêtre (*Beechwood creosote*) | 0,5 |
| Ethyle méthyle pyridine | 12,6 |
| Acide thioglycolique | 0,7 |
| Acide pyroligneux | 12 |
| Essence de goudron de bouleau rectifiée | 30,6 |
| Dipropylène Glycol | 38,5 |

Pour chaque test, trois échantillons sont préparés :
- un premier échantillon témoin constitué de la mauvaise odeur seule,
- un second échantillon témoin constitué de la composition parfumée seule, et
- un troisième échantillon constitué d'un mélange de la mauvaise odeur et de la composition parfumée.

L'échantillon témoin constitué de la composition parfumée seule est présenté sur mouillette, à la libre disposition des panélistes. Les produits parfumés concentrés sont testés à hauteur de 0,15 g sur un morceau de cellulose de 4 cm² qui est introduit dans un premier bocal en verre inodore d'une contenance de 5 L.

L'échantillon témoin constitué de la mauvaise odeur seule est réalisé de la façon suivante : la mauvaise odeur est introduite dans un second bocal en verre inodore d'une contenance de 5 L sur un morceau de cellulose de 4 cm² (80µg pour la mauvaise odeur de cuisine, 10 µg pour la mauvaise odeur de toilettes et 50µg pour la mauvaise odeur de tabac).

L'échantillon constitué d'un mélange de la mauvaise odeur et de la composition parfumée est réalisé de la façon suivante :
- la mauvaise odeur est introduite dans un troisième bocal en verre inodore d'une contenance de 5 L sur un morceau de cellulose de 4 cm² (80µg pour la mauvaise odeur de cuisine, 10 µg pour la mauvaise odeur de toilettes et 50µg pour la mauvaise odeur de tabac).
- les produits parfumés concentrés sont testés à hauteur de 0,15 g sur un morceau de cellulose de 4 cm². Ce morceau de cellulose est disposé à côté de celui de la mauvaise odeur.

Les bocaux sont codés, randomisés et anonymisés selon la norme ASTM E1958-12 afin de garantir un test en aveugle et ainsi minimiser les biais de perceptions conscientes du sujet testé. Les bocaux sont laissés 30 min avant de débuter les tests d'évaluation afin que le parfum et la mauvaise odeur se diffusent.

### Evaluation du test

L'évaluation est réalisée par le Panel Expert Interne (Parfumeurs, Evaluateurs et Analystes) et comporte en moyenne 15 personnes. Le panel évalue la composition parfumée puis remplit un questionnaire en monadique séquentiel, i.e. chaque expert teste un produit A, le note, puis teste un produit B, sans toutefois faire intervenir une comparaison consciente directe. L'intensité de la mauvaise odeur et l'intensité du parfum du troisième échantillon sont ainsi comparées avec les deux échantillons témoins. Les intensités sont notées sur une échelle linéaire de 1 (Très faible) à 10 (très puissant).

### Analyse statistique des données :

Le traitement statistique des données s'effectue avec une analyse de la variance (Intervalle de confiance 95%) grâce au logiciel FIZZ by BIOSYSTEMES.

Les produits sont discriminés à l'aide d'une lettre A, B, C etc. en cas de significativité et par NC en cas de non significativité.

### Résultats

### - Utilisation des compositions No. 1 et 2

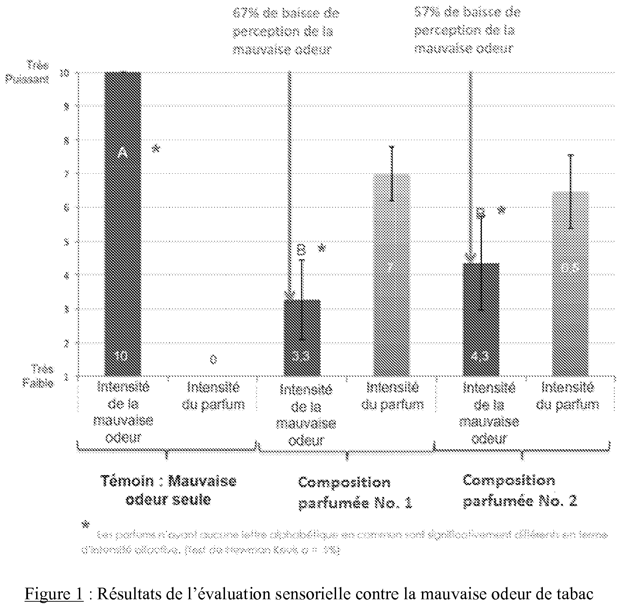

Les compositions parfumées No. 1 et 2, analysées indépendamment dans le cas d'une mauvaise odeur de tabac, ont permis de diminuer la perception olfactive de la mauvaise odeur de tabac respectivement de 67% et de 57% (voir figure No. 1).

### - Utilisation des compositions No. 3 et 4

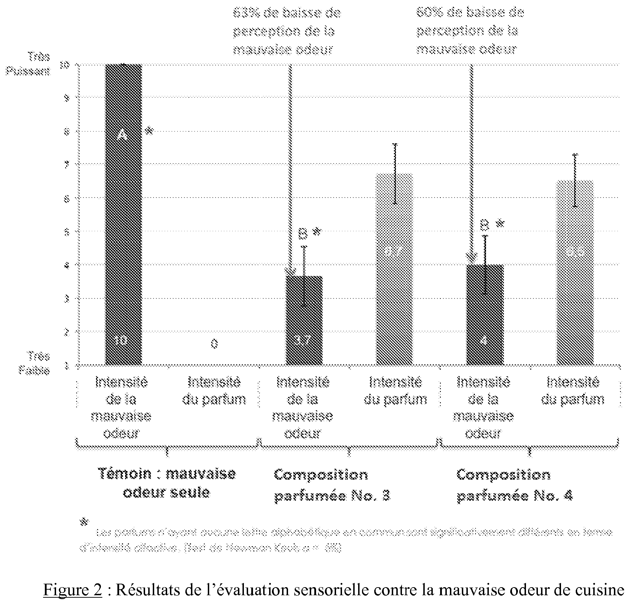

Les compositions parfumées No. 3 et 4, analysées indépendamment dans le cas d'une mauvaise odeur de cuisine, ont permis de diminuer la perception olfactive de la mauvaise odeur de cuisine respectivement de 63% et de 60% (voir figure No. 2).

### - Utilisation des compositions No. 5 et 6

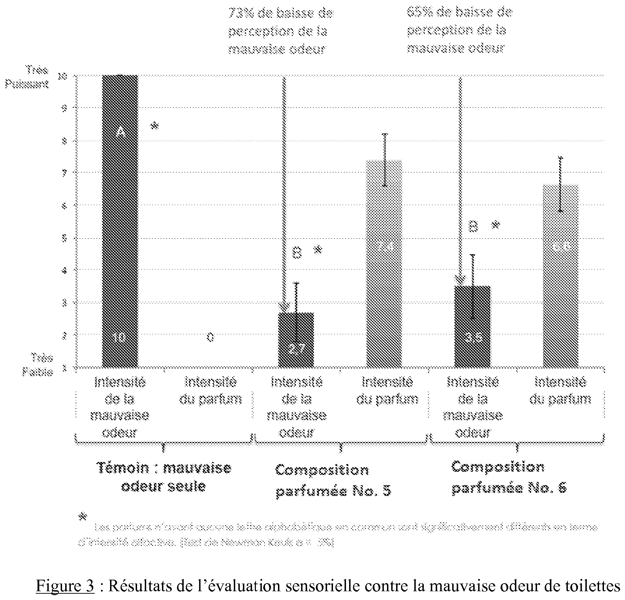

Les compositions parfumées No. 5 et 6, analysées indépendamment dans le cas d'une mauvaise odeur de toilette, ont permis de diminuer la perception olfactive de la mauvaise odeur de toilettes respectivement de 73% et de 65% (voir figure No. 3).

Les compositions parfumées étudiées permettent de diminuer de façon significative la perception des mauvaises odeurs de toilette, cuisine et de tabac. Le mélange masquant et les compositions parfumées selon l'invention permettent donc de masquer olfactivement les mauvaises odeurs.

### Exemple 8:

Les composants du tableau 10 ont été mélangés afin d'obtenir le mélange masquant N°7 comparatif.

**Tableau 10**

| **Désignation du produit** | **% En poids** |
|---|---|
| **2,4-diméthyl-4-phényltetrahydrofurane (rhubafurane)** | **50** |
| **Bicyclononalactone** | **50** |

Les composants du tableau 11 ont été mélangés afin d'obtenir le mélange masquant N°8 selon la présente invention.

**Tableau 11**

| **Désignation du produit** | **% En poids** |
|---|---|
| **2,4-diméthyl-4-phényltetrahydrofurane (rhubafurane)** | **35** |
| **Dihydro-5-pentyl-2(3H)-furanone (gamma-nonalactone)** | **35** |
| **Ester éthylique de l'acide 10-undécénoïque (undécylénate d'éthyle)** | **30** |

### Exemple 9 :

Le mélange masquant N°7 (comparatif) et les autres composants de la composition parfumée No. 9 (comparative), décrits dans le tableau 12, sont mélangés afin d'obtenir la composition parfumée No. 9 (comparative).

**Tableau 12**

| **Désignation du produit** | **% En poids** |
|---|---|
| di-isobutyl carbinyl acetate (Alicate®) | 16.67 |
| 3-(1,3-benzodioxol-5-yl)-2-methylpropanal (Aquanal ®, Helional ®) | 8.33 |
| 1-(2,3,8,8-tetraméthyl-1,2,3,4,5,6,7,8-octahydronaphthalen-2-yl)ethanone (Iso Ambois®) | 16.67 |
| Mousse de chêne (moss oakmoss synthetic) | 1.67 |
| Diluant (dipropylène glycol) | 53.32 |
| **Mélange masquant No 7 (comparatif)** | **3.34** |

Le mélange masquant No. 8 et les autres composants de la composition parfumée No. 10 (comparative), décrits dans le tableau 13, sont mélangés afin d'obtenir la composition parfumée No. 10 (comparative).

**Tableau 13**

| **Désignation du produit** | **% En poids** |
|---|---|
| di-isobutyl carbinyl acetate (Alicate®) | 16.67 |
| 3-(1,3- benzodioxol-5-yl)-2-methylpropanal (Aquanal ®, Helional ®) | 8.33 |
| 1-(2,3,8,8-tetraméthyl-1,2,3,4,5,6,7,8-octahydronaphthalen-2-yl)ethanone (Iso Ambois®) | 16.67 |
| Mousse de chêne (moss oakmoss synthetic) | 1.67 |
| Diluant (dipropylène glycol) | 53.32 |
| **Mélange masquant No. 8** | **3.34** |

### Exemple 10 :

Le mélange masquant No. 7 (comparatif) et les autres composants de la composition parfumée No. 11, décrits dans le tableau 14, sont mélangés afin d'obtenir la composition parfumée No. 11.

**Tableau 14**

| **Désignation du produit** | **% En poids** |
|---|---|
| Acétate de (2E)-3,7-diméthylocta-2,6-dièn-1-yle | 0,063 |
| [1R,(1R*,4E,9S*)]-4,11,11-triméthyl-8-méthylènebicyclo[7.2.0]undec-4-ène | 0,125 |
| Dodécanal | 0,188 |
| Endo-1,7,7-triméthyl-bicyclo-[2.2.1]heptan-2-ol | 0,375 |
| Acétate de 3,7-diméthyl-6-octèn-1-ol | 0,500 |
| Hexan-1-ol | 0,563 |
| Huile de feuille de Rosmarinus officinalis | 0,625 |
| Ester 2-propénylique de l'acide (3-methylbutoxy)-acétique | 0,688 |
| Octan-2-one | 0,875 |
| Huile de Lavandula Hybride (Lavandula Hybrida) | 1,250 |
| Huile de feuille de menthe verte (Mentha viridis) | 1,250 |
| Acétate de (4-méthyl-1-propan-2-yl-1-cyclohex-2-enyl) | 1,375 |
| Acétate d'hexyle | 1,563 |
| 2H-1-benzopyran-2-one | 1,625 |
| 1,7,7-triméthylbicyclo[2.2.1]-2-heptanone | 2,000 |
| Huile de feuille d'Eucalyptus globulus | 2,500 |
| Acétate de 3,7-diméthyl-octa-1,6-diène-3-yle | 3,125 |
| 3,7-diméthyl-octa-1,6-diène-3-ol | 3,750 |
| (1R, 2S, 5R)-5-méthyl-2-(1-méthyléthyl)-cyclohexanol | 3,750 |
| Acétate de 3a,4,5,6,7,7a-hexahydro-4,7-méthanoindèn-6-yle | 4,000 |
| Acétate de 4-tert-butylcyclohexyle | 4,000 |
| Acétate d'alpha,alpha,4-triméthylcyclohexylméthyle | 4,375 |
| Exo-acétate de bicyclo[2.2.1]heptan-2-ol-1,7,7-triméthyle | 5,000 |
| 2,6-diméthyloct-7-èn-2-ol | 5,250 |
| Huile de Cèdre de Texas (Juniperus Mexicana) | 6,250 |
| 1,1'-oxydipropan-2-ol | 43,935 |
| **Mélange masquant No 7 (comparatif)** | **1,000** |

Un mélange masquant No. 8 et les autres composants de la composition parfumée No. 12, décrits dans le tableau 15, sont mélangés afin d'obtenir la composition parfumée No. 12.

**Tableau 15**

| **Désignation du produit** | **% En poids** |
|---|---|
| Acétate de (2E)-3,7-diméthylocta-2,6-dièn-1-yle | 0,063 |
| [1R,(1R*,4E,9S*)]-4,11,11-triméthyl-8-méthylènebicyclo[7.2.0]undec-4-ène | 0,125 |
| Dodécanal | 0,188 |
| Endo-1,7,7-triméthyl-bicyclo-[2.2.1]heptan-2-ol | 0,375 |
| Acétate de 3,7-diméthyl-6-octèn-1-ol | 0,500 |
| Hexan-1-ol | 0,563 |
| Huile de feuille de Rosmarinus officinalis | 0,625 |
| Ester 2-propénylique de l'acide (3-methylbutoxy)-acétique | 0,688 |
| Octan-2-one | 0,875 |
| Huile de Lavandula Hybride (Lavandula Hybrida) | 1,250 |
| Huile de feuille de menthe verte (Mentha viridis) | 1,250 |
| Acétate de (4-méthyl-1-propan-2-yl-1-cyclohex-2-enyl) | 1,375 |
| Acétate d'hexyle | 1,563 |
| 2H-1-benzopyran-2-one | 1,625 |
| 1,7,7-triméthylbicyclo[2.2.1]-2-heptanone | 2,000 |
| Huile de feuille d'Eucalyptus globulus | 2,500 |
| Acétate de 3,7-diméthyl-octa-1,6-diène-3-yle | 3,125 |
| 3,7-diméthyl-octa-1,6-diène-3-ol | 3,750 |
| (1R, 2S, 5R)-5-méthyl-2-(1-méthyléthyl)-cyclohexanol | 3,750 |
| Acétate de 3a,4,5,6,7,7a-hexahydro-4,7-méthanoindèn-6-yle | 4,000 |
| Acétate de 4-tert-butylcyclohexyle | 4,000 |
| Acétate d'alpha,alpha,4-triméthylcyclohexylméthyle | 4,375 |
| Exo-acétate de bicyclo[2.2.1]heptan-2-ol-1,7,7-triméthyle | 5,000 |
| 2,6-diméthyloct-7-èn-2-ol | 5,250 |
| Huile de Cèdre de Texas (Juniperus Mexicana) | 6,250 |
| 1,1'-oxydipropan-2-ol | 43,935 |
| **Mélange masquant No 8** | **1,000** |

### Test de réduction des mauvaises odeurs :

### Des tests similaires à ceux de l'exemple 7 ont été réalisés.

### Résultats :

### - Utilisation des compositions No. 9 et 12.

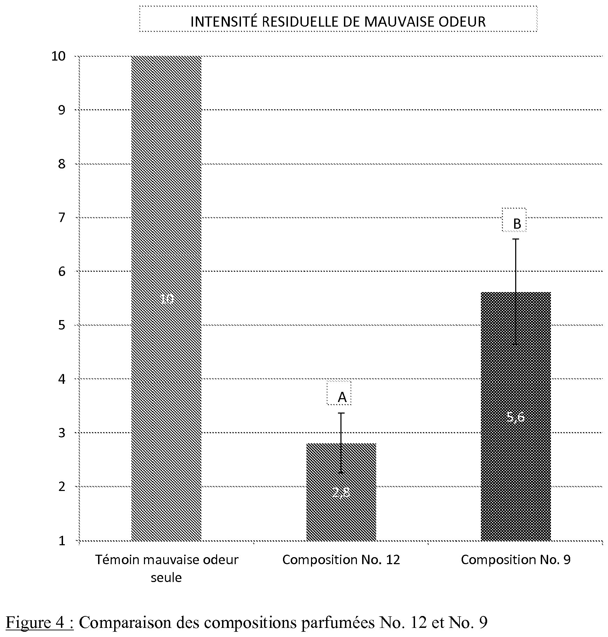

Les compositions parfumées No 9 et 12, analysées indépendamment dans le cas d'une mauvaise odeur de toilettes, ont permis de diminuer la perception olfactive de la mauvaise odeur de toilettes respectivement de 44% et 72% (voir figure No. 4). La composition parfumée selon la présente invention (No. 12) est donc plus efficace que la composition parfumée comparative (No. 9).

### - Utilisation des mélanges masquants No. 7 et 8.

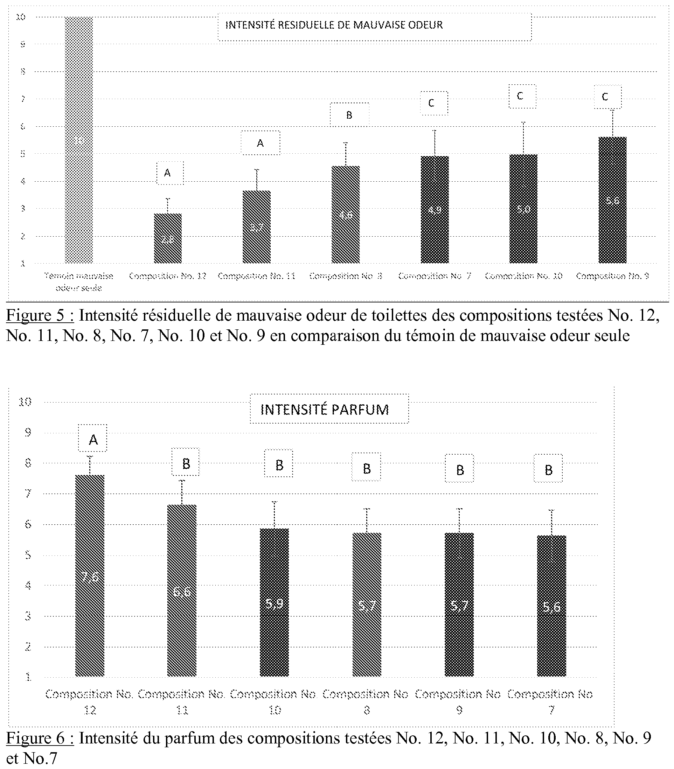

Les mélanges masquants No. 7 et 8, analysés indépendamment dans le cas d'une mauvaise odeur de toilettes, ont permis de diminuer la perception olfactive de la mauvaise odeur de toilettes respectivement de 51% et 54% (voir figure No. 5). Le mélange masquant selon la présente invention à base de gamma-nonlactone (No. 8) est donc plus efficace que le mélange masquant comparatif à base de bicyclononalactone (No. 7). La gamma-nonalactone est donc plus performante que la bicyclononalactone.

### - Utilisation des compositions No. 11 et 12.

Les compositions parfumées No 11 et 12, analysées indépendamment dans le cas d'une mauvaise odeur de toilettes, ont permis de diminuer la perception olfactive de la mauvaise odeur de toilettes respectivement de 63% et 72% (voir figure No. 5).

La composition parfumée selon la présente invention à base de gamma-nonalactone (No. 12) est donc plus efficace que la composition parfumée comprenant le mélange masquant comparatif à base de biclyclononalactone (No. 11). Dans une composition parfumée selon l'invention, l'ajout du mélange masquant à base de gamma-nonalactone est donc plus efficace que l'ajout d'un mélange masquant à base de bicyclononalactone. La gamma-nonalactone est donc plus performante que la bicyclononalactone.

### - Utilisation des compositions parfumée No. 9 et 10.

Les compositions parfumées No 9 et 10, analysées indépendamment dans le cas d'une mauvaise odeur de toilettes, ont permis de diminuer la perception olfactive de la mauvaise odeur de toilettes respectivement de 44% et 50% (voir figure No. 5). Dans une composition parfumée de l'art antérieur, l'ajout du mélange masquant selon la présente invention à base de gamma-nonalactone (composition parfumée No. 10) est plus efficace que l'ajout d'un mélange masquant comparatif à base de bicyclononalactone (composition parfumée No. 9). La gamma-nonalactone est donc plus performante que la bicyclononalactone.

### - Utilisation des compositions No. 9 et 12.

Les compositions parfumées No 9 et 12, analysées indépendamment montrent que la composition parfumée selon la présente invention (No. 12) présente une intensité de parfum significativement supérieure à la composition parfumée comparative (No. 9) (voir figure No. 6).

### - Utilisation des compositions No. 11 et 12.

Les compositions parfumées No 11 et 12, analysées indépendamment montrent que la composition parfumée selon la présente invention (No. 12) présente une intensité de parfum significativement supérieure à la composition parfumée comparative (No. 11) (voir figure No. 6).

Les compositions parfumées selon la présente invention permettent de diminuer de façon significative la perception des mauvaises odeurs testées et présentent une meilleure efficacité en comparaison avec les compositions de l'art antérieur. Le mélange masquant et les compositions parfumées selon l'invention permettent donc de mieux masquer olfactivement les mauvaises odeurs en comparaison avec d'autres compositions de l'art antérieur. Outre, l'efficacité contre les mauvaises odeurs, les compositions parfumées selon l'invention présentent une meilleure puissance olfactive (intensité de parfum) en comparaison avec les compositions parfumées comparatives de l'art antérieur.

## Revendications

1. Mélange comprenant de la dihydro-5-pentyl-2(3H)-furanone à un pourcentage compris entre 20 et 80% par rapport au poids total dudit mélange et du 2,4-diméthyl-4-phényltétrahydrofurane à un pourcentage compris entre 20 et 80% par rapport au poids total dudit mélange.

2. Mélange selon la revendication 1, **caractérisé en ce que** la dihydro-5-pentyl-2(3H)-furanone représente entre 30 et 35% par rapport au poids total dudit mélange et le 2,4-diméthyl-4-phényltétrahydrofurane représente entre 30 et 35% par rapport au poids total dudit mélange.

3. Mélange selon la revendication **1 ou 2, caractérisé en ce qu'**il comprend en outre de l'ester éthylique de l'acide 10-undécénoïque et/ou de la (3E)-4-(2,6,6-triméthylcyclohex-2-èn-1-yl)but-3-èn-2-one.

4. Mélange selon la revendication **3, caractérisé en ce que** la concentration d'ester éthylique de l'acide 10-undécénoïque est comprise entre 20 et 75% par rapport au poids total dudit mélange et la concentration de la (3E)-4-(2,6,6-triméthylcyclohex-2-èn-1-yl)but-3-èn-2-one est comprise entre 20 et 75% par rapport au poids total dudit mélange.

5. Mélange selon la revendication **4, caractérisé en ce que** la concentration d'ester éthylique de l'acide 10-undécénoïque est comprise entre 30 et 35% par rapport au poids total dudit mélange et la concentration de la (3E)-4-(2,6,6-triméthylcyclohex-2-èn-1-yl)but-3-èn-2-one est comprise entre 30 et 35% par rapport au poids total dudit mélange.

6. Composition parfumée comprenant le mélange tel que défini selon l'une quelconque des revendications **1 à 5.**

7. Base cosmétique comprenant le mélange tel que défini selon l'une quelconque des revendications **1 à 5.**

8. Base détergente comprenant le mélange tel que défini selon l'une quelconque des revendications **1 à 5.**

9. Parfum d'ambiance comprenant le mélange tel que défini selon l'une quelconque des revendications **1 à 5.**

10. Utilisation du mélange tel que défini selon l'une quelconque des revendications **1 à 5** pour préparer une composition cosmétique, une base détergente ou un parfum d'ambiance.

11. Utilisation du mélange tel que défini selon l'une quelconque des revendications **1 à 5,** de la composition parfumée telle que définie selon la revendication **6,** de la base cosmétique telle que définie selon la revendication **7,** de la base détergente telle que définie selon la revendication **8** ou du parfum d'ambiance tel que défini selon la revendication **9,** pour masquer les mauvaises odeurs.

## Patentansprüche

1. Mischung, die Dihydro-5-Pentyl-2(3H)-Furanon entsprechend einem Prozentsatz, der zwischen 20 und 80% in Bezug auf das Gesamtgewicht der Mischung enthalten ist, und 2,4-Dimethyl-4-Phenyltetrahydrofuran entsprechend einem Prozentsatz, der zwischen 20 und 80% in Bezug auf das Gesamtgewicht der Mischung enthalten ist, umfasst.

2. Mischung nach Anspruch 1, **dadurch gekennzeichnet, dass** das Dihydro-5-Pentyl-2(3H)-Furanon zwischen 30 und 35% in Bezug auf das Gesamtgewicht der Mischung repräsentiert und das 2,4-Dimethyl-4-Phenyltetrahydrofuran zwischen 30 und 35% in Bezug auf das Gesamtgewicht der Mischung repräsentiert.

3. Mischung nach Anspruch 1 oder 2, **dadurch gekennzeichnet, dass** sie weiter Ethylester der 10-Undecensäure und/ oder des (3E)-4-(2,6,6-Trimethylcyclohex-2-en-1-yl)but-3-en-2-on umfasst.

4. Mischung nach Anspruch 3, **dadurch gekennzeichnet, dass** die Ethylester-Konzentration der 10-Undecensäure zwischen 20 und 75% in Bezug auf das Gesamtgewicht der Mischung enthalten ist und die Konzentration von 3E)-4-(2,6,6-Trimethylcyclohex-2-en-1-yl)but-3-en-2-on zwischen 20 und 75% in Bezug auf das Gesamtgewicht der Mischung enthalten ist.

5. Mischung nach Anspruch 4, **dadurch gekennzeichnet, dass** die Ethylester-Konzentration der 10-Undecensäure zwischen 30 und 35% in Bezug auf das Gesamtgewicht der Mischung enthalten ist und die Konzentration von 3E)-4-(2,6,6-Trimethylcyclohex-2-en-1-yl)but-3-en-2-on zwischen 30 und 35% in Bezug auf das Gesamtgewicht der Mischung enthalten ist.

6. Parfümierte Zusammensetzung, die die Mischung nach einem der Ansprüche 1 bis 5 umfasst.

7. Kosmetische Grundlage, die die Mischung nach einem der Ansprüche 1 bis 5 umfasst.

8. Reinigungsmittelgrundlage, die die Mischung nach einem der Ansprüche 1 bis 5 umfasst.

9. Raumduft, der die Mischung nach einem der Ansprüche 1 bis 5 umfasst.

10. Verwendung der Mischung nach einem der Ansprüche 1 bis 5, zur Herstellung einer kosmetischen Zusammensetzung, einer Reinigungsmittelgrundlage oder eines Raumduftes.

11. Verwendung der Mischung nach einem der Ansprüche 1 bis 5, der parfümierten Zusammensetzung nach Anspruch 6, der kosmetischen Grundlage nach Anspruch 7, der Reinigungsmittelgrundlage nach Anspruch 8 oder des Raumduftes nach Anspruch 9 zur Maskierung unangenehmer Gerüche.

## Claims

1. Mixture comprising dihydro-5-pentyl-2(3H)-furanone at a percentage of between 20 and 80% relative to the total weight of said mixture and 2,4-dimethyl-4-phenyltetrahydrofuran at a percentage of between 20 and 80% relative to the total weight of said mixture.

2. Mixture according to claim 1, **characterised in that** the dihydro-5-pentyl-2(3H)-furanone represents between 30 and 35% relative to the total weight of said mixture and the 2,4-dimethyl-4-phenyltetrahydrofuran represents between 30 and 35% relative to the total weight of said mixture.

3. Mixture according to claim 1 or 2, **characterised in that** it further comprises 10-undecenoic acid ethyl ester and/or (3E)-4-(2,6,6- trimethylcyclohex-2-en-1-yl)but-3-en-2-one.

4. Mixture according to claim 3, **characterised in that** the concentration of 10-undecenoic acid ethyl ester is between 20 and 75% relative to the total weight of said mixture and the concentration of the (3E)-4-(2,6,6-trimethylcyclohex-2-en-1-yl)but-3-en-2-one is between 20 and 75% relative to the total weight of said mixture.

5. Mixture according to claim 4, **characterised in that** the concentration of 10-undecenoic acid ethyl ester is between 30 and 35% relative to the total weight of said mixture and the concentration of the (3E)-4-(2,6,6-trimethylcyclohex-2-en-1-yl)but-3-en-2-one is between 30 and 35% relative to the total weight of said mixture.

6. Fragranced composition comprising the mixture as defined according to any of claims 1 to 5.

7. Cosmetic base comprising the mixture as defined according to any of claims 1 to 5.

8. Detergent base comprising the mixture as defined according to any of claims 1 to 5.

9. Ambient fragrance comprising the mixture as defined according to any of claims 1 to 5.

10. Use of the mixture as defined according to any of claims 1 to 5 for preparing a cosmetic composition, a detergent base or an ambient fragrance.

11. Use of the mixture as defined according to any of claims 1 to 5, of the fragranced composition as defined according to claim 6, of the cosmetic base as defined according to claim 7, of the detergent base as defined according to claim 8 or of the ambient fragrance as defined according to claim 9, for masking unpleasant odours.
